# FASCICULE DE BREVET EUROPEEN

(11) **EP 1 221 912 B1**
(45) Date de publication et mention de la délivrance du brevet: **31.08.2005**
(21) Numéro de dépôt: 00969636.0
(22) Date de dépôt: 18.10.2000
(51) Int. Cl.: A61F 2/44

(54) **PROTHESE DE DISQUE INTERVERTEBRAL A FACES DENTEES**
BANDSCHEIBENPROTHESE MIT ZÄHNEN AUF DEN SEITEN
INTERVERTEBRAL DISK PROSTHESIS WITH TOOTHED SURFACES

(30) Priorité: 18.10.1999 FR 9912951
(43) Date de publication de la demande: 17.07.2002
(73) Titulaire: Stryker Spine, 33610 Cestas (FR)
(72) Inventeur: BACCELLI, Christian, F-33640 Ayguemorte les Graves (FR); SENEGAS, Jacques, 33700 Merignac (FR); ZEIGER, Evan, Birmingham, AL 35234 (US); BARROS, Tarcisio, University of Sao Paolo, 01332 Sao Paolo, SP (BR); SALGER, Dietmar, D-19067 Retgendorf (DE); VAN DEN EEDEN, Franck, NL-242 BV Massenar (NL)
(74) Mandataire: Texier, Christian
(86) Numéro de dépôt international: PCT/FR2000/002899
(87) Numéro de publication internationale: WO 2001/028463

(56) Documents cités:
- WO-A-99/38461
- WO-A1-00/13618
- WO-A1-00/44320
- WO-A1-01/03615
- WO-A1-01/15637
- FR-A- 2 726 759
- FR-A- 2 782 914
- US-A- 5 192 327
- US-A- 5 865 845

## Description

L'invention concerne les prothèses de disque intervertébral.

On connaît des prothèses de disque intervertébral présentant sur leurs deux faces principales des dents allongées parallèlement les unes aux autres. De telles dentures assurent un bon ancrage de la prothèse entre les plateaux des vertèbres adjacentes à l'espace de disque occupé par la prothèse.

Une prothèse selon le préambule de la revendication 1 est décrite dans le document FR-2 727 003.

Des prothèses de disque intervertébral présentant deux faces principales supérieure et inférieure opposées l'une à l'autre et dentées, ces faces montrant, dans au moins un plan transversal, un profil de forme générale non rectiligne sont divulguées dans les documents WO 99 38461 et US 5 865 845.

Un but de l'invention est de fournir une prothèse assurant un ancrage encore meilleur.

En vue de la réalisation de ce but, on prévoit selon l'invention une prothèse conforme à la revendication 1.

Ainsi, on peut choisir le profil de la face dentée pour qu'il soit le plus possible complémentaire de celui du plateau vertébral avec lequel cette face est destinée à venir en contact. Alors que la prothèse de l'art antérieur pouvait nécessiter de préparer la face du plateau vertébral pour modifier sa forme en vue d'améliorer le contact avec la prothèse, une telle préparation devient la plupart du temps inutile concernant la face dentée de la prothèse selon l'invention. Cela réduit la durée de l'intervention chirurgicale nécessaire pour installer la prothèse. La face, dentée et conformée au plateau, permet un ancrage de très grande qualité contre le plateau vertébral.

De plus, une face dentée dont le profil présente deux tronçons de forme générale rectiligne inclinés l'un par rapport à l'autre est particulièrement adaptée à la forme des plateaux de certaines vertèbres.

Avantageusement, l'un des deux tronçons a une longueur au moins double de celle de l'autre tronçon.

La face a ainsi un profil en aile d'avion correspondant de très près à la forme de certains plateaux vertébraux.

Avantageusement, la face dentée présente une zone formant l'un des tronçons et dépourvue de dents.

Avantageusement, la face dentée présente une zone dépourvue de dents et contiguë à un bord de la face.

Avantageusement, la prothèse présente un évidement central s'étendant de l'une à l'autre des faces principales.

Un tel évidemment peut par exemple recevoir du greffon facilitant l'intégration osseuse de la vertèbre.

Avantageusement, la prothèse comporte une paroi latérale présentant au moins un orifice s'étendant d'une face externe de la paroi à l'évidement central.

Ainsi, cet orifice accroît la vascularisation du greffon logé dans l'évidement.

Avantageusement, la prothèse présente une face latérale constituée par une première portion cylindrique s'étendant sur plus de 180° par référence à un axe du cylindre, et une deuxième portion cylindrique de plus grand rayon que la première portion.

Une telle forme permet à la prothèse d'occuper la plus grande partie de la surface des plateaux vertébraux.

Avantageusement, les dents ont un profil incliné d'un même côté de la prothèse.

Cette forme facilite l'introduction de la prothèse entre les plateaux mais constitue un frein à son retrait.

Avantageusement, la prothèse comporte au moins un picot s'étendant en saillie dé l'une des faces principales, notamment de la face dentée.

Ainsi, le picot s'implante dans le plateau vertébral et accroît encore la qualité de l'ancrage.

Avantageusement, la prothèse présente un orifice fileté.

Cet orifice peut coopérer avec un outil de pose de la prothèse pour sa fixation à la prothèse. Il peut également servir à relier la prothèse à une plaque fixée latéralement aux corps vertébraux.

Avantageusement, il s'agit d'une prothèse apte à être utilisée pour un disque cervical.

On prévoit également selon l'invention un ensemble comprenant une prothèse selon l'invention et un outil de pose de celle-ci.

Avantageusement, la prothèse et l'outil comportent des moyens de fixation de la prothèse à l'outil dans une position relative prédéterminée et des moyens de détrompage agencés de sorte que cette position est unique.

D'autres caractéristiques et avantages de l'invention apparaîtront encore dans la description suivante d'un mode préféré de réalisation et d'une variante donnés à titre d'exemples non limitatifs. Aux dessins annexés :
- les figures 1 à 5 sont des vues respectives en perspective, de dessus, de face, arrière et de droite d'une prothèse selon un mode préféré de réalisation de l'invention ;
- les figures 6 et 7 sont des vues en coupe d'une vertèbre cervicale respectivement suivant les plans sagittal et frontal médians ;
- les figures 8 et 9 sont des vues en perspective de la prothèse de la figure 1 avec son outil de pose ; et
- la figure 10 est une vue partielle analogue à la figure 4 illustrant une variante de réalisation.

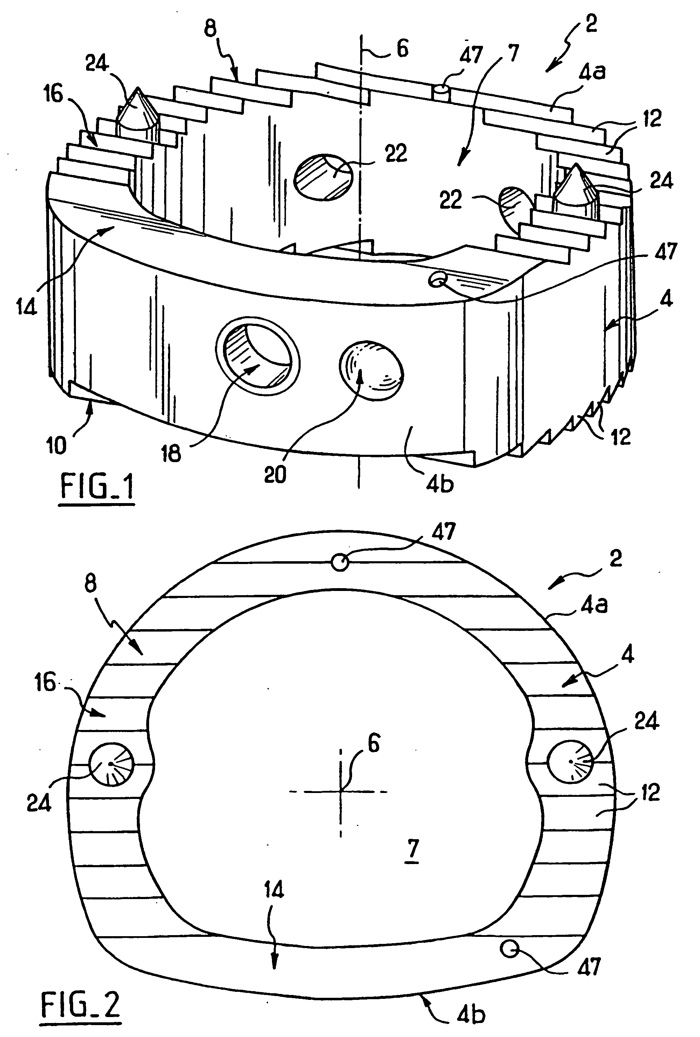

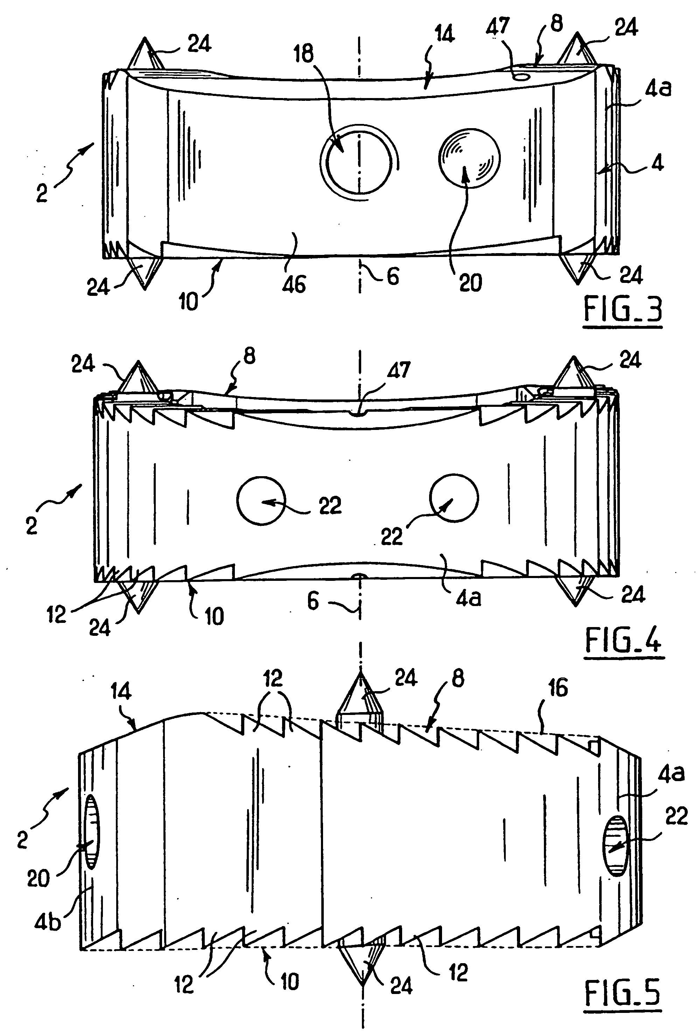

En référence aux figures 1 à 5, la prothèse 2 est ici une cage présentant une paroi 4 de forme générale annulaire d'axe 6.

En vue de dessus, la paroi comporte une première portion 4a en forme de fer à cheval. La portion 4a a une forme cylindrique d'axe 6. Elle s'étend sur environ 250° autour de l'axe 6. La paroi comporte une deuxième portion 4b également de forme cylindrique, s'étendant sur environ 20° autour de son axe qui n'est pas l'axe 6 mais est parallèle à celui-ci. La deuxième portion 4b a un rayon beaucoup plus grand que celui de la première portion 4a. Les extrémités de la première portion rejoignent celles de la deuxième portion. La cage a donc en plan la forme d'une couronne un peu aplatie sur un côté 4b qui est ici le côté antérieur ou avant de la cage. Cette paroi 4 définit au centre de la cage un évidement 7.

La cage présente deux faces principales supérieure 8 et inférieure 10 opposées l'une à l'autre et s'étendant généralement dans des plans parallèles entre eux et perpendiculaires à l'axe 6. L'évidement 7 s'étend de l'une à l'autre des deux faces 8, 10.

La face inférieure 10 est en l'espèce précisément perpendiculaire à l'axe 4. Elle présente dans un plan sagittal, c'est-à-dire parallèle à l'axe 4 et perpendiculaire à la paroi avant 4b, un profil denté formant des dents allongées 12 parallèles entre elles et parallèles à la paroi avant 4b. Toutes les dents 12 sont identiques entre elles et ont notamment même hauteur. Les dents sont toutes inclinées vers l'avant. Elles présentent un flanc avant parallèle à l'axe 6 et un flanc arrière incliné.

Dans le plan sagittal précité, la face supérieure 8 a un profil constitué de deux tronçons de forme générale rectiligne 14, 16 inclinés l'un par rapport à l'autre pour donner au profil une forme convexe. Le tronçon arrière 16 est le plus long des deux tronçons. Il s'étend, dans ce plan, sur environ 80% de la longueur de la cage. Le tronçon a un profil denté. Toutes les dents 12 sont identiques entre elles et notamment ont même hauteur. Elles sont inclinées vers l'avant comme les dents de la face inférieure 10. Le tronçon 16 est légèrement incliné vers l'arrière de la cage. Il est donc légèrement incliné par rapport à la face inférieure 10.

Le tronçon avant 14 est incliné vers l'avant de la cage plus fortement que l'inclinaison du tronçon arrière vers l'arrière. Sa longueur est environ égale au quart de celle du tronçon arrière 16. Le tronçon avant 14 est lisse. Il correspond à la partie de la face supérieure s'étendant sur la paroi avant 4b. Les deux tronçons 14, 16 donnent à la face supérieure 8 un profil proche de celui d'une aile d'avion.

La cage comporte un orifice fileté de montage et de fixation 18, s'étendant dans le plan sagittal médian de la prothèse, dans la paroi avant 4b et mettant en communication la face externe de cette dernière avec l'évidement central 7. D'un côté de cet orifice 18, par exemple sur le côté droit, la face externe de la paroi avant présente une cavité hémisphérique 20 servant au détrompage, comme on le verra plus loin.

Sur la partie de la paroi la plus en arrière, la cage comporte deux orifices 22 mettant eux aussi la face externe en communication avec l'évidement central 7. Les deux orifices 22 sont disposés symétriquement par rapport au plan sagittal médian de la cage.

La cage comporte des picots 24, ici au nombre de 4, soit deux pour chaque face principale associée 8, 10. Chaque picot a une extrémité pointue et s'étend en saillie de la face principale associée. Les deux picots de chaque face sont disposés symétriquement l'un de l'autre par rapport au plan sagittal médian. De plus, ils s'étendent dans le plan frontal médian passant par l'axe 6. Chaque picot d'une face s'étend au droit d'un picot de l'autre face.

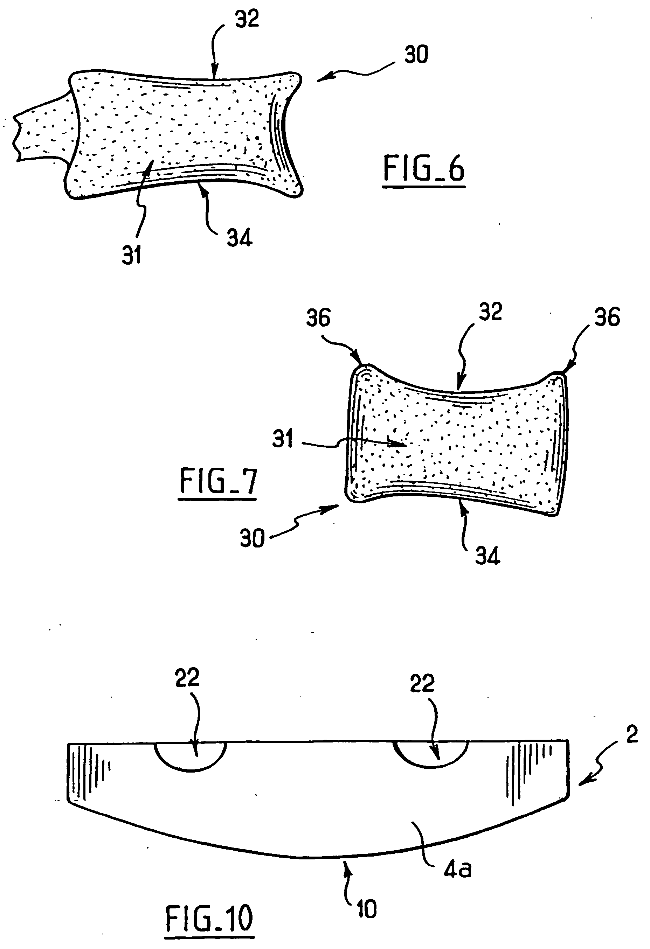

La cage que l'on vient de décrire est particulièrement adaptée à occuper un espace intervertébral cervical. On a illustré aux figures 6 et 7 un corps vertébral 31 d'une vertèbre cervicale 30. En coupe suivant le plan sagittal médian à la figure 6, le plateau supérieur 32 de la vertèbre a un profil sensiblement rectiligne horizontal alors que le plateau inférieur 34 a un profil concave en aile d'avion, complémentaire de celui convexe de la face supérieure 8 de la cage. En coupe suivant un plan frontal médian, en référence à la figure 7, le plateau inférieur 34 a un profil sensiblement rectiligne horizontal alors que le plateau supérieur 32 a un profil courbe concave dont les sommets sont les incus 36.

Lorsque la cage est insérée entre deux vertèbres cervicales 31, les faces principales 8, 10 de la cage épousent donc très étroitement la forme des plateaux 32, 34 avec lesquels elles viennent en contact.

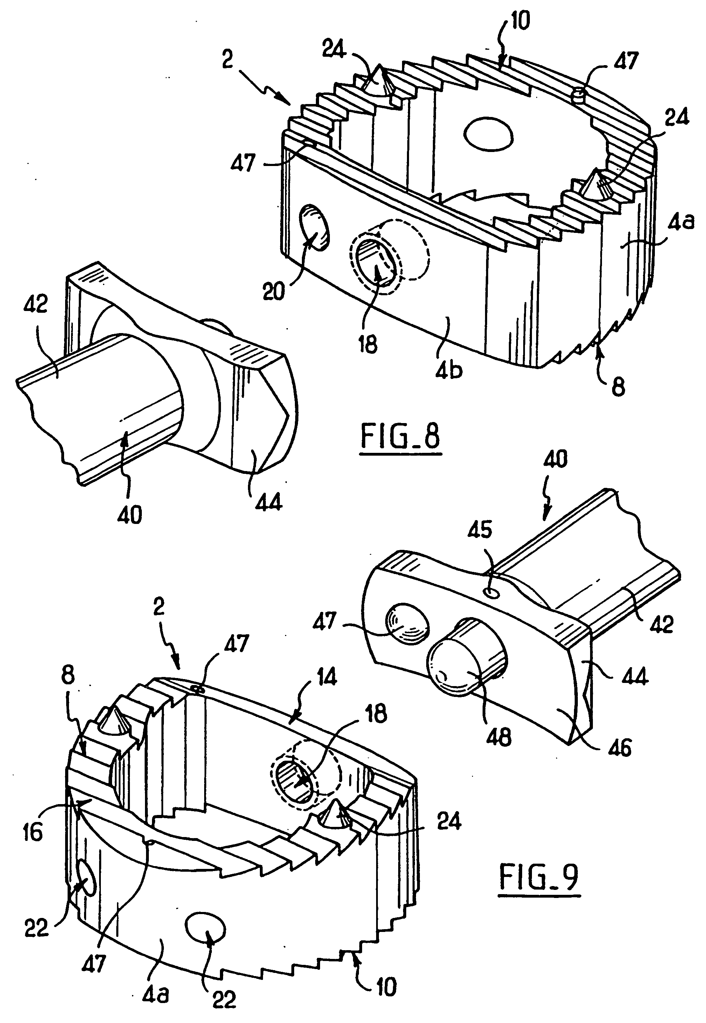

Pour mettre en place la cage, on pourra avantageusement utiliser un outil de pose 40 tel que celui des figures 8 et 9. Cet outil comporte un corps 42 présentant une tête 44 ayant une face avant 46 de forme rectangulaire, dont la forme et la hauteur sont proches de celles de la face avant de la cage 2. L'outil comporte un embout fileté 48 émergeant au centre de la face 46 de la tête, mobile par rapport à celle-ci et manoeuvrable depuis une autre extrémité de l'outil. Cet embout est apte à former une liaison filetée avec l'orifice de montage 18 de la cage. La face avant 46 de la tête comporte un relief sphérique 47 en saille apte à pénétrer dans la cavité sphérique avant 20 de la cage lorsque l'embout 48 est relié à l'orifice 18. Le relief 47 et la cavité 20 forment des moyens de détrompage. Grâce à eux, il existe une seule position de fixation de la cage 2 à l'outil 40 avec les contours de la face avant de la cage en coïncidence avec ceux de la tête 44 de l'outil.

Pour mettre en place la cage entre les vertèbres, on opère une distraction des vertèbres par des moyens appropriés afin d'agrandir la hauteur de l'espace de disque entre ces vertèbres. On prépare le plateau supérieur 32 de la vertèbre inférieure 10 pour rapprocher sa forme de celle de la face inférieure 10 de la cage. L'évidement 7 reçoit avant montage du greffon osseux ou toute autre substance permettant la croissance osseuse. La cage étant montée sur l'outil, on met en place la cage entre les vertèbres par la voie antérieure. Puis on interrompt la distraction. Les plateaux vertébraux 32, 34 viennent ainsi en appui sur les faces principales 8, 10 de la cage. Les picots 24 s'ancrent dans ces plateaux. L'orientation des dents 12, après avoir facilité l'insertion de la cage, limite les possibilités d'avancée de la cage hors de son logement. La cage ne nécessite pas une préparation du plateau inférieur 34 de la vertèbre supérieure.

Dans une variante de réalisation illustrée à la figure 10, la face inférieure de la cage, encore dentée (sans que cela ait été illustré) a en coupe parallèle à un plan frontal, une forme courbe convexe en arc de cercle. Une telle cage ne nécessite pas la préparation du plateau supérieur 32 de la vertèbre inférieure de sorte qu'aucun plateau vertébral n'a besoin d'être préparé. Une telle cage s'adapte donc au mieux à l'anatomie de l'espace intervertébral.

L'orifice de montage 18 pourra être utilisé pour fixer la cage à un système d'ancrage, par exemple une plaque antérieure ancrée par ailleurs dans des faces latérales des corps vertébraux 31.

Les trois orifices 18, 22 facilitent la vascularisation du greffon reçu dans l'évidement 7.

Pour accroître encore le détrompage, la tête 44 de l'outil peut porter un marquage 45 permettant de reconnaître la face supérieure 8 de la cage lorsque celle-ci est fixée à l'outil dans la bonne position.

La cage pourra être en matériau radiotransparent, par exemple en PEEK (Poly-Ether-Ether-Ketone). Dans ce cas, la cage pourra comprendre un ou plusieurs marqueurs 47 inclus dans la cage et permettant du fait de leur radioopacité, de pouvoir repérer la position et/ou la présence de l'implant lors de radiographies per- ou postopératoires. Ils pourront être en titane ou alliage de titane. Les marqueurs 47 sont ici au nombre de deux et formées par des fils insérés dans des conduits rectilignes parallèles à l'axe 6, ménagés dans la paroi de la cage. L'un des conduits s'étend à l'arrière, dans le plan sagittal médian, et l'autre à l'extrémité gauche de la paroi avant.

Les picots 24 pourront être insérés et fixés rigidement dans des conduits ménagés dans la cage. Ils pourront eux aussi être réalisés dans un matériau radioopaque.

## Revendications

1. Prothèse (2) de disque intervertébral, présentant deux faces principales supérieure (8) et inférieure (10) opposées l'une à l'autre, au moins la face supérieure présentant des dents profilées (12) parallèles les unes aux autres, **caractérisée en ce que** la face supérieure dentée (8) a, dans au moins un plan transversal à cette face, un profil de forme générale non rectiligne, le profil de la face supérieure dentée (8) présentant deux tronçons de forme générale rectiligne (14, 16) inclinés l'un par rapport à l'autre.

2. Prothèse selon la revendication 1, **caractérisée en ce que** l'un (16) des deux tronçons a une longueur au moins double de celle de l'autre tronçon (14).

3. Prothèse selon l'une quelconque des revendications 1 ou 2, **caractérisée en ce que** la face dentée présente une zone (14) formant l'un des tronçons et dépourvue de dents.

4. Prothèse selon l'une quelconque des revendications 1 à 3, **caractérisée en ce que** la face dentée (8) présente une zone (14) dépourvue de dents et contiguë à un bord de la face.

5. Prothèse selon l'une quelconque des revendications 1 à 4, **caractérisée en ce qu'**elle présente un évidement central (7) s'étendant de l'une à l'autre des faces principales (8, 10).

6. Prothèse selon la revendication 5, **caractérisée en ce qu'**elle comporte une paroi latérale présentant au moins un orifice (18, 22) s'étendant d'une face externe de la paroi à l'évidement central (7).

7. Prothèse selon l'une quelconque des revendications 1 à 6, **caractérisée en ce qu'**elle présente une face latérale (4) constituée par une première portion cylindrique (4a) s'étendant sur plus de 180° par référence à un axe (6) du cylindre, et une deuxième portion cylindrique (4b) de plus grand rayon que la première portion (4a).

8. Prothèse selon l'une quelconque des revendications 1 à 7, **caractérisée en ce que** les dents (12) ont un profil incliné d'un même côté de la prothèse.

9. Prothèse selon l'une quelconque des revendications 1 à 8, **caractérisée en ce qu'**elle comporte au moins un picot (24) s'étendant en saillie de l'une des faces principales (8, 10), notamment de la face dentée.

10. Prothèse selon l'une quelconque des revendications 1 à 9, **caractérisée en ce qu'**elle présente un orifice fileté (18).

11. Prothèse selon l'une quelconque des revendications 1 à 10, **caractérisée en ce qu'**il s'agit d'une prothèse apte à être utilisée pour un disque cervical.

12. Ensemble **caractérisé en ce qu'**il comprend une prothèse (2) selon l'une des revendications 1 à 11 et un outil (40) de pose de cette prothèse.

13. Ensemble selon la revendication 12, **caractérisé en ce que** la prothèse (2) et l'outil (40) comportent des moyens de fixation (18, 48) de la prothèse à l'outil dans une position relative prédéterminée et des moyens de détrompage (20, 47) agencés de sorte que cette position est unique.

## Patentansprüche

1. Bandscheibenprothese (2), die zwei Hauptflächen aufweist, nämlich eine obere (8) und eine untere (10), die einander gegenüberliegen, wobei mindestens die obere Fläche profilierte Zähne (12) aufweist, die zueinander parallel sind, **dadurch gekennzeichnet, daß** die obere, gezahnte Fläche (8) in mindestens einer, zu dieser Fläche querverlaufenden Ebene ein Profil mit insgesamt nicht geradliniger Form hat, wobei das Profil der oberen, gezahnten Fläche (8) zwei Stücke (14, 16) mit insgesamt geradliniger Form aufweist, die zueinander geneigt sind.

2. Prothese nach Anspruch 1, **dadurch gekennzeichnet, daß** das eine (16) der beiden Stücke eine Länge von mindestens dem Doppelten der des anderen Stückes (14) hat.

3. Prothese nach einem der Ansprüche 1 oder 2, **dadurch gekennzeichnet, daß** die gezahnte Fläche eine Zone (14) aufweist, die das eine der Stücke bildet und keine Zähne aufweist:

4. Prothese nach einem der Ansprüche 1 bis 3, **dadurch gekennzeichnet, daß** die gezahnte Fläche (8) eine Zone (14) aufweist, die keine Zähne aufweist und an einen Rand der Fläche angrenzt.

5. Prothese nach einem der Ansprüche 1 bis 4, **dadurch gekennzeichnet, daß** sie eine mittige Aussparung (7) aufweist, die sich von der einen der Hauptflächen (8, 10) zur anderen erstreckt.

6. Prothese nach Anspruch 5, **dadurch gekennzeichnet, daß** sie eine seitliche Wand umfaßt, die mindestens eine Öffnung (18, 22) aufweist, die sich von einer Außenfläche der Wand zur mittigen Aussparung (7) erstreckt.

7. Prothese nach einem der Ansprüche 1 bis 6, **dadurch gekennzeichnet, daß** sie eine Seitenfläche (4) aufweist, die von einem ersten, zylindrischen Abschnitt (4a), der sich über mehr als 180° erstreckt, bezogen auf die Zylinderachse (6), und einem zweiten, zylindrischen Abschnitt (4b) mit größerem Radius als der erste Abschnitt (4a) gebildet ist.

8. Prothese nach einem der Ansprüche 1 bis 7, **dadurch gekennzeichnet, daß** die Zähne (12) ein Profil haben, das zu ein und, derselben Seite der Prothese geneigt ist.

9. Prothese nach einem der Ansprüche 1 bis 8, **dadurch gekennzeichnet, daß** sie mindestens eine Spitze (24) aufweist, die von einer der Hauptflächen (8, 10), besonders von der gezahnten Fläche, vorspringt.

10. Prothese nach einem der Ansprüche 1 bis 9, **dadurch gekennzeichnet, daß** sie eine mit Gewinde versehene Öffnung (18) aufweist.

11. Prothese nach einem der Ansprüche 1 bis 10, **dadurch gekennzeichnet, daß** es sich um eine Prothese handelt, die geeignet ist, als Hals-Bandscheibe verwendet zu werden.

12. Anordnung, **dadurch gekennzeichnet, daß** sie eine Prothese (2) nach einem der Ansprüche 1 bis 11 sowie ein Werkzeug (40) zum Einsetzen dieser Prothese umfaßt.

13. Anordnung nach Anspruch 12, **dadurch gekennzeichnet, daß** die Prothese (2) und das Werkzeug (40) Mittel (18, 48) zur Befestigung der Prothese am Werkzeug in einer bestimmten Relativlage sowie Mittel zur Polarisierung (20, 47) umfassen, die derart eingerichtet sind, daß diese Lage einzigartig ist.

## Claims

1. An intervertebral disk prosthesis (2) having two main faces, a superior main face (8) and an inferior main face (10) that are opposite to each other, at least the superior face having mutually parallel shaped teeth (12), the prosthesis being **characterized** it that the general shape of the profile of the superior toothed face (8) in at least one plane extending transversely to said face is not rectilinear, the profile of the superior toothed face (8) presenting two segments (14, 16) of generally rectilinear shape that are inclined relative to each other.

2. A prosthesis according to claim 1, **characterized in that** one of the two segments (16) is at least twice as long as the other segment (14).

3. A prosthesis according to claim 1 or 2, **characterized in that** the toothed face has a zone (14) that forms one of the segments, and that has no teeth.

4. A prosthesis according to any one of claims 1 to 3, **characterized in that** the toothed face (8) has a zone (14) that is without teeth and that is contiguous with an edge of the face.

5. A prosthesis according to any one of claims 1 to 4, **characterized in that** it has a central hole (7) extending from one of the main faces (8, 10) to the other.

6. A prosthesis according to claim 5, **characterized in that** it has a side wall presenting at least one orifice (18, 22) extending from an outer face of the wall to the central hole (7).

7. A prosthesis according to any one of claims 1 to 6, **characterized in that** it has a side face (4) constituted by a first cylindrical portion (4a) extending over more than 180° about a cylinder axis (6), and a second cylindrical portion (4b) of greater radius than the first portion (4a).

8. A prosthesis according to any one of claims 1 to 7, **characterized in that** the teeth (12) have a profile that slopes towards the same side of the prosthesis.

9. A prosthesis according to any one of claims 1 to 8, **characterized in that** it has at least one spike (24) projecting from one of its main faces (8, 10), in particular from its toothed face.

10. A prosthesis according to any one of claims 1 to 9, **characterized in that** it has a tapped orifice (18).

11. A prosthesis according to any one of claims 1 to 10, **characterized in that** it is a prosthesis suitable for being used for a cervical disk.

12. A kit, **characterized in that** it comprises a prosthesis (2) according to any one of claims 1 to 11, and a tool (40) for fitting said prosthesis.

13. A kit according to claim 12, **characterized in that** the prosthesis (2) and the tool (40) have fixing means (18, 48) for fixing the prosthesis to the tool in a predetermined relative position, and keying means (20, 47) organized to ensure that there is only one such position.
